(19) Europäisches Patentamt

European Patent Office

Office européen des brevets

(11) EP 1 575 931 B1

(12) EUROPÄISCHE PATENTSCHRIFT

(45) Veröffentlichungstag und Bekanntmachung des Hinweises auf die Patenterteilung:
09.08.2006 Patentblatt 2006/32

(21) Anmeldenummer: 03782378.8

(22) Anmeldetag: 12.12.2003

(51) Int Cl.:
*C07D 323/06* (2006.01)

(86) Internationale Anmeldenummer:
PCT/EP2003/014122

(87) Internationale Veröffentlichungsnummer:
WO 2004/054998 (01.07.2004 Gazette 2004/27)

(54) **VERFAHREN ZUR HERSTELLUNG VON TRIOXAN**

METHOD FOR THE PRODUCTION OF TRIOXANE

PROCEDE POUR PRODUIRE DU TRIOXANE

(84) Benannte Vertragsstaaten:
AT BE BG CH CY CZ DE DK EE ES FI FR GB GR HU IE IT LI LU MC NL PT RO SE SI SK TR

(30) Priorität: 13.12.2002 DE 10258663

(43) Veröffentlichungstag der Anmeldung:
21.09.2005 Patentblatt 2005/38

(73) Patentinhaber: Ticona GmbH
65451 Kelsterbach (DE)

(72) Erfinder:
• HAUBS, Michael
65545 Bad Kreuznach (DE)
• GÖRING, Matthias
65719 Hofheim (DE)
• HOFFMOCKEL, Michael
65527 Niedernhausen (DE)
• LINGNAU, Jürgen
55130 Mainz (DE)
• MÜCK, Karl-Friedrich
65207 Wiesbaden (DE)

(56) Entgegenhaltungen:
EP-A- 0 133 669      EP-A- 0 148 293
EP-A- 0 484 786      US-A- 3 483 214
US-A- 4 340 542

Bemerkungen:
Die Akte enthält technische Angaben, die nach dem Eingang der Anmeldung eingereicht wurden und die nicht in dieser Patentschrift enthalten sind.

EP 1 575 931 B1

**Beschreibung**

Die Erfindung betrifft ein Verfahren zur Herstellung von 1,3,5-Trioxan (im folgenden Trioxan genannt) aus wässrigen Formaldehydlösungen in Gegenwart von Säure als Katalysator mit hoher Selektivität und geringem Energieaufwand.

Die Herstellung von Trioxan aus wässrigen Formaldehydlösungen ist seit langem bekannt {vgl. z.B. US-A-3,483,214). Nach dem Stand der Technik wird Trioxan aus konzentrierten wäßrigen Formaldehydlösungen in Gegenwart saurer Katalysatoren gebildet. Durch Destillation der Reaktionsmischung wird eine trioxanreiche Gasphase gewonnen. Durch weitere, bekannte Trennverfahren läßt sich daraus reines Trioxan herstellen.

Die DE-A-1,543,390 beschreibt ein Verfahren, bei dem der aus dem Reaktor austretende trioxanhaltige Dampf in einer Kolonne einem weitgehend ausreagierten Reaktionsgemisch entgegengeführt wird. Besonders vorteilhaft wird das Reaktionsgemisch aus dem Reaktor dem trioxanhaltigen Dampf entgegengeführt. Als Ausgangsmaterial wird Formaldehyd als konzentrierte wässrige Lösung dem Reaktor zugeführt.

Aus der DE-A-4,035,495 ist ein Verfahren zur Herstellung von Trioxan bekannt, bei dem Acetalpolymere in Gegenwart saurer Katalysatoren abgebaut werden. Das Verfahren wird in einem bekannten Umlaufreaktor mit Verdampfer durchgeführt.

Die genannten Verfahren sind jedoch in folgenden Punkten verbesserungswürdig:

1. Bildung von Nebenprodukten im Reaktor
2. Energieaufwand beim Destillieren der Reaktionsmischung
3. Korrosion im Reaktor bzw. in der Kolonne durch den Katalysator

Aufgabe der Erfindung war es, das Verfahren zur Herstellung von Trioxan bezüglich der genannten Punkte 1. - 3. zu verbessern.

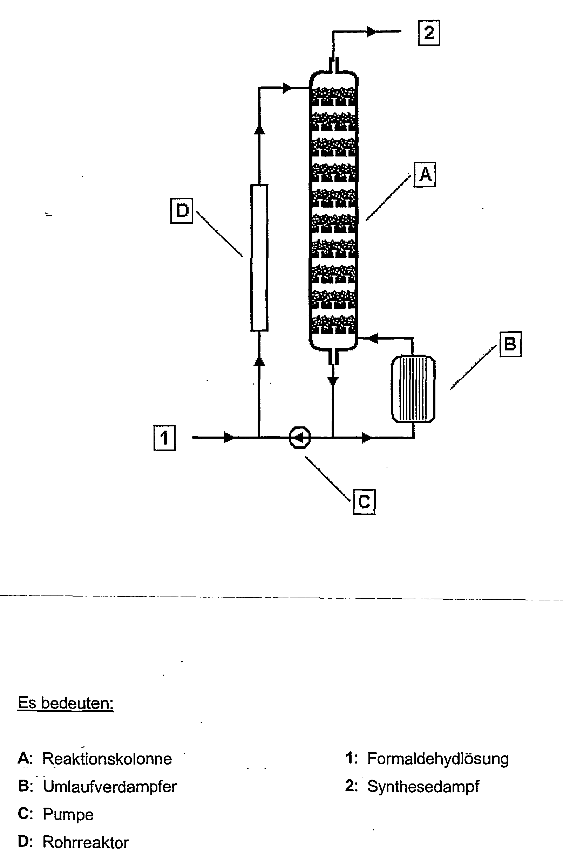

Die Lösung der Aufgabe sei anhand Abb. 1 erläutert:
Die Reaktionsmischung aus wässriger Formaldehydlösung und Katalysator befindet sich in der Reaktionskolonne **A** und füllt deren hold-up-Volumen aus. Aus dem Sumpfstrom der Kolonne **A** wird mittels des Umlaufverdampfers **B** ein Dampfgemisch erzeugt, mit dem die Reaktionskolonne **A** von unten beaufschlagt wird. Ein Teil des Sumpfstromes wird mit frischer Formaldehydlösung **1** gemischt und mit Hilfe der Pumpe **C** über den Rohrreaktor **D** als Rücklaufstrom dem oberen Teil der Reaktionskolonne **A** zugeführt. Der trioxanhaltige Synthesedampf **2** wird gasförmig als Kopfprodukt der Kolonne abgezogen.

Wie aus Abb.1 ersichtlich und im weiteren Text ausführlich beschrieben, wird dem Rohrreaktor ein Gemisch zugeführt, das im wesentlichen aus frischer, aufkonzentrierter Formaldehyd-Lösung besteht. Dadurch lassen sich bei niedriger Katalysatorkonzentration eine hohe Trioxankonzentration und eine hohe Raum-Zeitausbeute erreichen. Durch die Verwendung einer geringen Katalysatorkonzentration wird die korrosive Wirkung des Katalysators verringert; durch die hohe Trioxankonzentration wird die Trioxankonzentration im Synthesedampf erhöht und damit der Energieverbrauch verringert und schließlich wird durch die hohe Raum-Zeitausbeute bei niedriger Katalysatorkonzentration die Bildung von Nebenprodukten zurückgedrängt.

Als Reaktionskolonne eignen sich alle bekannten Konstruktionen. Sie müssen jedoch aus einem Material gefertigt sein, das densauren Reaktionsbedingungen widersteht. Geeignete Materialien sind z.B. Nickelbasislegierungen, Tantal oder Zirkonium. Auch kunststoffbeschichtete Kolonnen sind grundsätzlich geeignet.

Die Reaktionskolonne ist ferner durch ihr hold-up-Volumen $V_K$ charakterisiert. Bevorzugt werden Konstruktionen mit besonders hohem hold-up-Volumen, da das Volumen $V_K$ einen wesentlichen Teil des gesamten Reaktionsvolumens darstellt. Geeignet sind daher Kolonnen mit Glockenböden oder mit Siebböden mit hohen Wehren.

Als Rohrreaktor eignen sich Gefäße, einfache Rohre oder Rohrbündel. Um ein einheitliches Verweilzeitspektrum zu erzielen, können in dem Rohrreaktor statische Mischer oder als Mischer wirkende Verschlaufungen eingebaut sein.

Es hat sich als herausgestellt, daß die in Abb. 1 gezeigte Anlage besonders vorteilhaft betrieben werden kann, wenn folgende Parameter eingehalten werden: Sei $V_{FF}$ das pro Zeiteinheit dem Rohrreaktor zugeführte Volumen an frischer Formaldehydlösung und $V_{SV}$ das pro Zeiteinheit dem Rohrrektor zugeführte Kolonnensumpfvolumen, so liegt das Verhältnis $V_{FF} / V_{SV}$ erfindungsgemäß zwischen 0,5 und 20, bevorzugt zwischen 1 und 10 und besonders bevorzugt zwischen 2 und 5.

Der Umlaufverdampfer beinhaltet mit dem Sumpf der Kolonne ein bestimmtes Volumen, das Kolonnensumpfvolumen $V_{US}$. Es hat sich herausgestellt, daß das erfindungsgemäße Verfahren dann besonders gute Ergebnisse liefert, wenn das Volumen $V_{US}$ kleiner ist als das hold-up-Volumen der Reaktionskolonne. Sei $V_K$ das Reaktionsvolumen in der Kolonne, so liegt das Verhältnis $V_K / V_{US}$ erfindungsgemäß zwischen 1 und 10 und bevorzugt zwischen 2 und 5.

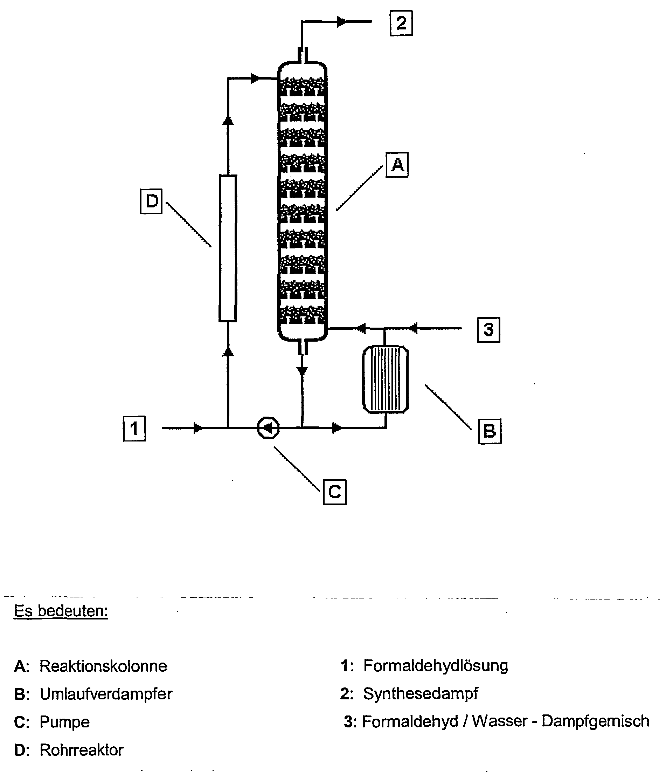

Die im Umlaufverdampfer erzeugte Dampfmenge richtet sich nach dem Eingangsstrom an wässriger Formaldehydlösung. Erfahrungsgemäß müssen pro kg einlaufender Formaldehydlösung etwa 0,7 bis 0,9 kg Dampf erzeugt werden. Der formaldehydhaltige Dampf, mit der die Kolonne im unteren Teil beaufschlagt wird, kann aber auch ganz oder teilweise von separaten Quellen, zum Beispiel aus anderen Anlageteilen, stammen. In diesem Fall kann die im Umlaufverdampfer erzeugte Dampfmenge entsprechend reduziert werden. Solche separaten dampfförmigen Formaldehyd-Quellen stehen im Aufarbeitungsteil einer technischen Trioxan-Anlage häufig zur Verfügung. Durch diese Formaldehyd-Dampf-Direktnutzung werden teure Kondensatoren eingespart, die Gesamtleistung der Anlage erhöht und die Prozeßkomplexität verringert. Diese vorteilhafie Ausführungsform der Erfindung ist in Abb. 2 schematisch dargestellt. Die Konzentration an Formaldehyd im Gemisch **3** liegt zwischen 35 Gew.% und 100 Gew.%, bevorzugt zwischen 45 Gew.% und 75 Gew.% und besonders bevorzugt zwischen 55 Gew.% und 65 Gew.%.

Die aus dem Rohrreaktor austretende Reaktionsmischung soll weitgehend ausreagiert sein, d.h. die Trioxankonzentration soll praktisch den Gleichgewichtswert erreicht haben. Das Volumen des Rohrreaktors wird so bemessen, daß die mittlere Verweilzeit im Rohrreaktor zur Gleichgewichtseinstellung ausreicht. Die dafür erforderliche mittlere Verweilzeit im Rohrreaktor hängt im wesentlichen von der Temperatur und der Katalysatorkonzentration, in etwas geringerem Ausmaß auch von der Formaldehydkonzentration ab. Erfahrungsgemäß liegt die mittlere Verweilzeit zwischen 1 min und 20 min, bevorzugt zwischen 2 min und 10 min. Die Temperatur am Ausgang des Rohrreaktors sollte etwa der Temperatur des obersten Bodens der Reaktionskolonne entsprechen.

Aus dem Sumpf der Reaktionskolonne wird zweckmäßigerweise ein kleiner Seitenstrom kontinuierlich oder absatzweise entnommen, um nichtflüchtige Verunreinigungen des Eingangsstromes sowie nichtflüchtige Nebenprodukte aus dem System zu entfernen.

Erfahrungsgemäß ist es ausreichend, wenn dieser Seitenstrom etwa 0,1% bis 1% des Eingangsstromes beträgt.

Die Konzentration der als Ausgangslösung verwendeten Formaldehydlösung liegt zwischen 50 Gew.% und 85 Gew.%, bevorzugt zwischen 60 Gew.% und 80 Gew.%. Die Temperatur der Lösung beträgt zwischen 60 °C und 150 °C, bevorzugt zwischen 70 und 130°C. Bei Temperaturen über 100°C muß die Lösung unter Überdruck gehalten werden, um ein Bilden von Dampfblasen zu verhindern.

Als Katalysatoren eignen sich starke Säuren, die gelöst oder ungelöst im Reaktionsgemisch vorliegen. Beispiele sind Schwefelsäure, Trifluormethansulfonsäure, Toluolsulfonsäure oder stark saure Ionenaustauscher. Auch saure Zeolithe oder Heteropolysäuren können verwendet werden. Die Konzentration an Katalysator liegt typischerweise zwischen 0,2 und 10 Gew.%, bevorzugt zwischen 0,4 Gew.% und 1,9 Gew.%. Unter den löslichen Katalysatoren wird Schwefelsäure bevorzugt.

Als ungelöste Katalysatoren werden handelsübliche, stark saure Ionenaustauscher bevorzugt. Sie können in der Reaktionsmischung suspendiert vorliegen oder in Form von Packungen eingesetzt werden.

Die Konzentration an Katalysator richtet sich nach der Konzentration der eingesetzten Formaldehydlösungen. Bei niedrigen Formaldehydkonzentrationen werden höhere Konzentrationen an Katalysator verwendet und umgekehrt. Wird Schwefelsäure als Katalysator verwendet, so liegt beispielsweise bei einer Formaldehydkonzentration von 60% die Schwefelsäurekonzentration bei 5 - 10 Gew.%, bei einer Formaldehydkonzentration von 80% liegt dagegen die Schwefelsäurekonzentration bei 0,2 bis 4 Gew.%.

Die (mittlere) Temperatur in der Reaktionskolonne hängt ebenfalls von der Konzentration der eingesetzten Formaldehydlösung ab. Mit steigender Formaldehydkonzentration steigt auch die Kolonnentemperatur. Sie liegt typischerweise zwischen 95 °C und 140 °C, bevorzugt zwischen 100 °C und 125 °C. Bei Temperaturen über 100°C wird die Kolonne unter Überdruck betrieben. Der Überdruck richtet sich nach der Formaldehydkonzentration und der Temperatur. Er liegt typischerweise im Bereich von etwa 0,1 bar bis 4 bar.

Der die Reaktionskolonne verlassende Synthesedampf enthält neben Trioxan auch noch Formaldehyd, Wasser und leichtflüchtige Nebenprodukte. Ausschlaggebend für den spezifischen Energieverbrauch ist die Trioxankonzentration im Synthesedampf. Beim Einsatz von 80 Gew.%-igen Formaldehydlösungen können Trioxankonzentrationen von bis zu 28 Gew.% erreicht werden.

Beispiele

Die folgenden Beispiele sollen die Erfindung erläutern, ohne sie zu beschränken.

Beispiel 1

Herstellung von Trioxan im Rohrreaktor:

Eine 79%ige wässrige Formaldehydlösung wird mit konzentrierter Schwefelsäure im Massenverhältnis von 100 : 1 gemischt und bei einer Temperatur von 120 °C in einen Rohrreaktor gegeben. Der Rohrreaktor besitzt in der Mitte und am Ende Vorrichtungen zur Probenahme. Die mittlere Verweilzeit wurde aus dem Volumen des Rohrreaktors und der Durchflussmenge gemäß folgender Formel berechnet:

$$t = V_{RR} \, / \, V_P$$

Es bedeuten:

t:     mittlere Verweilzeit in Minuten
$V_{RR}$:    Volumen des Rohrreaktors vom Anfang bis zur Probenahmestelle in Liter
$V_P$ :    Volumenstrom durch den Rohrreaktor in Liter / min.

Die genommenen Proben wurden mit verdünnter Lauge sofort neutralisiert, um eine nachträgliche Veränderung der Trioxankonzentration auszuschließen. Folgende Abhängigkeit der Trioxankonzentration von der Verweilzeit im Rohrreaktor wurde gemessen:

| Verweilzeit in Minuten : | 2 | 4 |
|---|---|---|
| Trioxankonzentration in Gew.-%: | 3,2 | 5,4 |

Bei einer Dichte der Reaktionslösung von 1,2 kg/Liter errechnet sich daraus eine Raum-Zeit-Ausbeute an Trioxan im Rohrreaktor von 770 kg/m$^3$/h bzw. von 650 kg/m$^3$/h.

Beispiel 2:

Der Versuch von Beispiel 1 wurde wiederholt, jedoch betrug die Konzentration an Schwefelsäure nur 0,5 Gew. %. Folgende Werte wurden gemessen:

| Verweilzeit in Minuten: | 3 | 6 |
|---|---|---|
| Trioxankonzentration in Gew%: | 3,1 | 5,2 |

Bei einer Dichte der Reaktionslösung von 1,2 kg/Liter errechnet sich daraus eine Raum-Zeit-Ausbeute an Trioxan im Rohrreaktor von 670 kg/m$^3$/h bzw. von 540 kg/m$^3$/h.

**Patentansprüche**

1. Verfahren zur Herstellung von Trioxan aus wässrigen Formaldehydlösungen in Gegenwart saurer Katalysatoren in einer Reaktionskolonne mit Umlaufverdampfer, **dadurch gekennzeichnet, daß** die als Ausgangslösung verwendete Formaldehydlösung mit einem Seitenstrom aus dem Sumpf der Reaktionskolonne gemischt und über einen Rohrreaktor dem oberen Teil der Reaktionskolonne zugeführt wird.

2. Verfahren nach Anspruch 1, **dadurch gekennzeichnet, daß** das Verhältnis von pro Zeiteinheit dem Rohrreaktor zugeführte Volumen an frischer Formaldehydlösung und dem pro Zeiteinheit dem Rohrreaktor zugeführte Volumen aus dem Sumpf der Reaktionskolonne zwischen 0,5 und 20, bevorzugt zwischen 1 und 10 und besonders bevorzugt zwischen 2 und 5 liegt.

3. Verfahren nach Anspruch 1, **dadurch gekennzeichnet, daß** das Volumen der Reaktionskolonne größer ist als das Kolonnensumpfvolumen.

4. Verfahren nach Anspruch 1, **dadurch gekennzeichnet, daß** die mittlere Verweilzeit im Rohrreaktor zwischen 1 min und 20 min, bevorzugt zwischen 2 min und 10 min. beträgt.

5. Verfahren nach Anspruch 1, **dadurch gekennzeichnet, dass** die Konzentration an saurem Katalysator unter 2 Gew.% liegt.

6. Verfahren Anspruch 1, **dadurch gekennzeichnet, daß** der Druck in der Reaktionskolonne über Atmosphärendruck und bevorzugt bei 1,5 bis 2,5 bar$_{abs}$ liegt.

**7.** Verfahren nach Anspruch 1, **dadurch gekennzeichnet, daß** der formaldehydhaltige Dampf, mit der die Kolonne im unteren Teil beaufschlagt wird, ganz oder teilweise aus separaten Quellen stammt.

**8.** Verfahren nach Anspruch 1, **dadurch gekennzeichnet, daß** als Katalysator stark saure Ionenaustauscher verwendet werden.

**9.** Vorrichtung zur Durchführung des Verfahrens nach Anspruch 1 umfassend

> i) eine Reaktionskolonne (A),
> ii) eine Ableitung für einen Sumpfstrom, die in
> iii) einen Umlaufverdampfer (B) mündet und diesem einen Teil des Sumpfstromes zuführt, wobei
> iv) von dem Umlaufverdampfer eine Ableitung für das erzeugte Dampfgemisch in den unteren Teil der Reaktionskolonne führt,
> v) aus Ableitung ii) vor deren Einmündung in den Umlaufverdampfer (B) eine Ableitung für die Abzweigung eines Teils des Sumpfstromes führt, die
> vi) mit einer Zuleitung für frische Formaldehydlösung in
> vii) einen Rohrreaktor (D) mündet, welcher über eine Ableitung
> viii) in den oberen Teil der Reaktionskolonne (A) mündet.

**10.** Vorrichtung nach Anspruch 9, **dadurch gekennzeichnet, dass** eine Pumpe (C) vorgesehen ist, mit der ein Teil des Sumpfstromes und der frischen Formaldehydlösung über den Rohrreaktor (D) dem oberen Teil der Reaktionskolonne (A) zugeführt wird.

**11.** Vorrichtung nach Anspruch 9, **dadurch gekennzeichnet, dass** mindestens eine Zuleitung vorgesehen ist, mit der formaldehydhaltiger Dampf, der nicht aus dem Umlaufverdampfer (B) stammt, dem unteren Teil der Reaktionskolonne (A) zugeführt werden kann.

**Claims**

**1.** A process for preparing trioxane from aqueous formaldehyde solutions in the presence of acidic catalysts in a reaction column equipped with a circulation evaporator, which comprises mixing the formaldehyde solution used as a starting solution with a sidestream from the bottom of the reaction column and feeding it to the upper section of the reaction column via a tubular reactor.

**2.** The process as claimed in claim 1, wherein the ratio of the volume per unit time of fresh formaldehyde solution fed to the tubular reactor and the volume per unit time from the bottom of the reaction column fed to the tubular reactor is between 0.5 and 20, preferably between 1 and 10 and more preferably between 2 and 5.

**3.** The process as claimed in claim 1, wherein the volume of the reaction column is greater than the column bottom volume.

**4.** The process as claimed in claim 1, wherein the average residence time in the tubular reactor is between 1 min and 20 min, preferably between 2 min and 10 min.

**5.** The process as claimed in claim 1, wherein the concentration of acidic catalyst is below 2% by weight.

**6.** The process claim 1, wherein the pressure in the reaction column is above atmospheric pressure and preferably from 1.5 to 2.5 $\text{bar}_{abs}$.

**7.** The process as claimed in claim 1, wherein some or all of the formaldehyde vapor with which the lower section of the column is charged stems from separate sources.

**8.** The process as claimed in claim 1, wherein the catalyst used is a strongly acidic ion exchanger.

**9.** An apparatus for carrying out the process as claimed in claim 1, comprising

> i) a reaction column (A),

ii) a bottom stream draw line which opens into

iii) a circulation evaporator (B) and feeds to it a portion of the bottom stream, and

iv) a draw line for the vapor mixture generated leads from the circulation evaporator into the lower section of the reaction column,

v) a draw line for the branching-off of a portion of the bottom stream leads from draw line ii) before it opens into the circulation evaporator (B) and,

vi) together with a feed line for fresh formaldehyde solution, opens into

vii) a tubular reactor (D) which opens via a draw line

viii) into the upper section of the reaction column (A).

10. The apparatus as claimed in claim 9, wherein a pump (C) is provided, with which a portion of the bottom stream and of the fresh formaldehyde solution is fed via the tubular reactor (D) to the upper section of the reaction column (A).

11. The apparatus as claimed in claim 9, wherein at least one feed line is provided, with which the formaldehyde-containing vapor which does not stem from the circulation evaporator (B) can be fed to the lower section of the reaction column (A).

**Revendications**

1. Procédé de fabrication du trioxanne à partir de solutions aqueuses de formaldéhyde en présence de catalyseurs acides dans une colonne de réaction à évaporateur à circulation, **caractérisé en ce que** la solution de formaldéhyde utilisée en tant que solution de départ est mélangée à un courant auxiliaire provenant du puits de la colonne de réaction et est acheminée, par l'intermédiaire d'un réacteur tubulaire, à la partie supérieure de la colonne de réaction.

2. Procédé selon la revendication 1, **caractérisé en ce que** le rapport du volume acheminé par unité de temps au réacteur tubulaire en solution fraîche de formaldéhyde et du volume acheminé par unité de temps au réacteur tubulaire en provenance du puits de la colonne de réaction se situe entre 0,5 et 20, de préférence, entre 1 et 10 et, en particulier, de préférence, entre 2 et 5.

3. Procédé selon la revendication 1, **caractérisé en ce que** le volume de la colonne de réaction est supérieur au volume du puits de la colonne.

4. Procédé selon la revendication 1, **caractérisé en ce que** la durée de séjour moyenne dans le réacteur tubulaire est comprise entre 1 minute et 20 minutes, de préférence, entre 2 minutes et 10 minutes.

5. Procédé selon la revendication 1, **caractérisé en ce que** la concentration en catalyseur acide se situe en dessous de 2% en poids.

6. Procédé selon la revendication 1, **caractérisé en ce que** la pression dans la colonne de réaction se situe au-dessus de la pression atmosphérique et, de préférence, à une valeur de 1,5 à 2,5 bars, en pression absolue.

7. Procédé selon la revendication 1, **caractérisé en ce que** la vapeur contenant du formaldéhyde, grâce à laquelle la colonne est chargée dans la partie inférieure, provient complètement ou partiellement de sources séparées.

8. Procédé selon la revendication 1, **caractérisé en ce que** l'on utilise, en tant que catalyseur, des résines échangeuses d'ions fortement acides.

9. Dispositif en vue de l'exécution du procédé selon la revendication 1, comprenant

i) une colonne de réaction (A)

ii) une dérivation pour un courant de puits,

iii) qui débouche dans un évaporateur à circulation (B) et qui achemine à celui-ci une partie du courant de puits,

iv) moyennant quoi une dérivation pour le mélange de vapeur produit conduit de l'évaporateur à circulation dans la partie inférieure de la colonne de réaction,

v) moyennant quoi une dérivation pour l'embranchement d'une partie du courant de puits conduit de la dérivation ii), avant son embouchure, dans l'évaporateur à circulation (B),

vi) qui débouche avec un conduit pour de la solution d'aldéhyde fraîche,

vii) dans un réacteur tubulaire (D), qui débouche,

viii) par l'intermédiaire d'un conduit, dans la partie supérieure de la colonne de réaction (A).

**10.** Dispositif selon la revendication 9, **caractérisé en ce que** l'on prévoit une pompe (C) grâce à laquelle une partie du courant de puits et de la solution de formaldéhyde fraîche est acheminée par l'intermédiaire du réacteur tubulaire (D) à la partie supérieure de la colonne de réaction (A).

**11.** Dispositif selon la revendication 9, **caractérisé en ce que** l'on prévoit au moins un conduit grâce auquel la vapeur contenant du formaldéhyde, qui ne provient pas de l'évaporateur à circulation (B), peut être acheminé à la partie inférieure de la colonne de réaction (A).

Es bedeuten:

A: Reaktionskolonne        1: Formaldehydlösung

B: Umlaufverdampfer       2: Synthesedampf

C: Pumpe

D: Rohrreaktor

**Es bedeuten:**

A: Reaktionskolonne

B: Umlaufverdampfer

C: Pumpe

D: Rohrreaktor

1: Formaldehydlösung

2: Synthesedampf

3: Formaldehyd / Wasser - Dampfgemisch